(19) Europäisches Patentamt
European Patent Office
Office européen des brevets

(11) **EP 2 037 307 A1**

(12) **EUROPEAN PATENT APPLICATION**
published in accordance with Art. 153(4) EPC

(43) Date of publication:
**18.03.2009 Bulletin 2009/12**

(51) Int Cl.:
**G02B 13/06** (2006.01)     **A61B 1/00** (2006.01)
**G02B 13/18** (2006.01)

(21) Application number: **07828118.5**

(22) Date of filing: **15.05.2007**

(86) International application number:
**PCT/JP2007/060306**

(87) International publication number:
**WO 2008/004377 (10.01.2008 Gazette 2008/02)**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **03.07.2006 JP 2006183291**

(71) Applicant: **Olympus Corporation
Tokyo 151-0072 (JP)**

(72) Inventor: **TOGINO, Takayoshi
Shibuya-Ku
Tokyo 151-0072 (JP)**

(74) Representative: **von Hellfeld, Axel
Wuesthoff & Wuesthoff
Patent- und Rechtsanwälte
Schweigerstrasse 2
81541 München (DE)**

(54) **OPTICAL SYSTEM**

(57)     The invention relates to an optical system that is capable of taking images having a wide angle-of-view in simplified construction, is of small-format size and well corrected for aberrations, and has high resolving power. The optical system is an imaging system that is to image an object plane (4) having a rotationally symmetric concave shape about a center axis (1) on a planar image plane (5) orthogonal to the center axis and comprises a refracting element (3) formed of a transparent medium including a first transmitting surface (31) rotationally symmetric about the center axis (1) and extending along said object plane (4) and a second transmitting surface (32) rotationally symmetric about the center axis (1) and having a refractive index of greater than 1. A light beam from the object plane (4) is imaged on the image plane (5) through the refracting element (3). The second transmitting surface (32) is concave on the image plane (5) side and has negative power at a position off the center axis (1) and in a section including the center axis (1).

FIG. 1

**Description**

ART FIELD

The present invention relates generally to an optical system, and more specifically to an objective or taking optical system adapted to form an image of a portion of an object having a large angle of view on a zonal image plane.

BACKGROUNF OF THE INVENTION

Wide-angle optical systems such as fisheye lenses have so far been used as means for taking images of peripheries having a wide angle of view. However, it has still been difficult to apply them to small-format optical instruments in general and endoscopes or capsule endoscopes in particular, because a lot more optical parts are required for taking images having a wide angle of view.

So far, the inner surface of a hemispherical transparent cover at the distal end of a capsule endoscope has been configured into a conical shape to achieve a wide-angle range of observation, as proposed in Patent Publication 1. However, what principles underlie remains unclear. Patent Publication 2 has proposed configuring the front surface of the transparent cover at the distal end of a capsule endoscope into a conical shape. However, nothing is still achieved about how this transparent cover is used for a wide angle-of-view arrangement.

Patent Publication 1
JP(A) 2001-174713
Patent Publication 2
United States Patent No. 5,604,531

DISCLOSURE OF THE INVENTION

Such being the situations of the prior art, a main object of the invention is to provide an optical system that is of small-format size and well corrected for aberrations, has high resolving power, and is capable of taking images having a wide angle of view in simplified construction.

According to the invention, the above object is achieved by the provision of an optical system which is an imaging system that is to image an object plane having a rotationally symmetric concave shape about a center axis on a planar image plane orthogonal to the center axis and comprises a refracting element formed of a transparent medium including a first transmitting surface rotationally symmetric about the center axis and extending along said object plane and a second transmitting surface rotationally symmetric about the center axis and having a refractive index of greater than 1, wherein a light beam from the object plane is imaged on the image plane through said refracting element, characterized in that said second transmitting surface is concave on an image plane side and has negative power at a position off the center axis and in a section including the center axis.

Desirously in this arrangement, a normal to said second transmitting surface makes an angle with the center axis near where the center axis intersects said second transmitting surface.

Desirously, said second transmitting surface is comprised of a toric surface rotationally symmetric about the center axis.

A normal to said first transmitting surface may make an angle with the center axis near where the center axis intersects said first transmitting surface.

Said second transmitting surface may be comprised of a spherical surface rotationally symmetric about the center axis.

It is desirous that the inventive optical system further comprises a rear unit that is located on an image plane side with respect to said refracting element, is rotationally symmetric about the center axis and has positive power, wherein a light beam from the object plane is imaged on the image plane through said refracting element and said rear unit in order.

It is desirous that between said refracting element and said rear unit or in said rear unit, there is an aperture located coaxially to the center axis.

It is desirous that a portion of an object image formed on the image plane that lies near the center axis is unavailable.

Astigmatism occurring at said refracting element may be corrected by an aspheric surface that is located on an opposite side thereto with said aperture interposed.

The invention also includes an endoscope comprising any one of the aforesaid optical systems, and a capsule endoscope comprising any one of the aforesaid optical systems as well, with said refracting element used as part of a domed transparent cover.

The invention also includes an optical system used to project an image located on the image plane onto the object plane.

According to the inventions as described above, it is possible to obtain an optical system that is of small-format size and well corrected for aberrations, has high resolving power, and is capable of taking or projecting images having a wide angle of view in simplified construction.

BRIEF DESCRIPTION OF THE DRAWINGS

Fig. 1 is a sectional view of the optical system according to Example 1 of the invention, as taken along the center axis.
Fig. 2 is a sectional view of one modification to the hemispherical inner surface (the second transmitting surface) of a transparent cover in Example 1.
Fig. 3 is a transverse aberration diagram for the optical system of Example 1.
Fig. 4 is a sectional view of the optical system according to Example 2 of the invention, as taken along the center axis.
Fig. 5 is a transverse aberration diagram for the optical system of Example 2.
Fig. 6 is a sectional view of the optical system according to Example 3 of the invention, as taken along the center axis.
Fig. 7 is a sectional view of one modification to the outer surface (the first transmitting surface) of a transparent cover in Example 3.
Fig. 8 is a transverse aberration diagram for the optical system of Example 3.
Fig. 9 is a sectional view of the optical system according to Example 4 of the invention, as taken along the center axis.
Fig. 10 is a transverse aberration diagram for the optical system of Example 4.
Fig. 11 is illustrative of an example of using the inventive wide angle-of-view taking optical system as a taking optical system at the distal end of an endoscope.
Fig. 12 is illustrative of one example of using the inventive wide angle-of-view taking optical system as a taking optical system for a capsule endoscope.

BEST MODE FOR CARRYING OUT THE INVENTION

The optical system of the invention is now explained with reference to some specific examples.

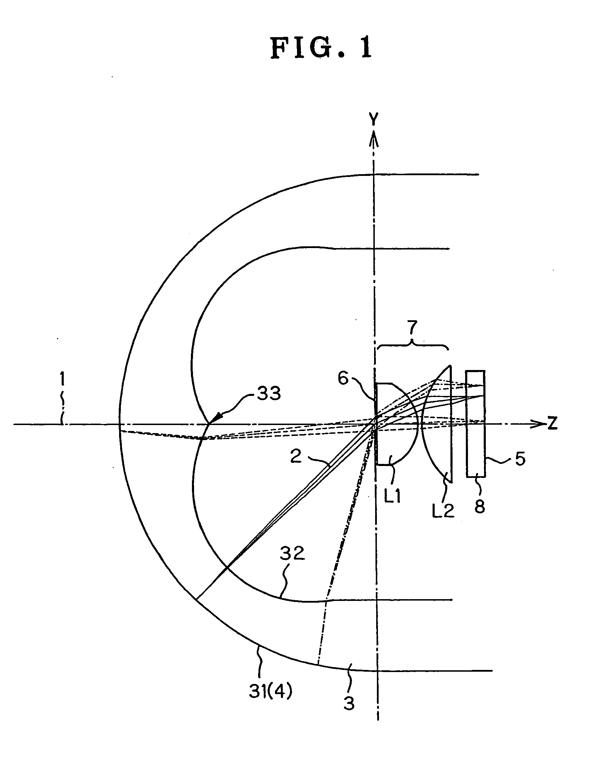

Fig. 1 is a sectional view of the optical system according to Example 1, as taken along the center axis 1 (the axis of rotational symmetry). The optical system of the invention is now explained more specifically with reference to an imaging optical system adapted to form an image on a curved object plane 4 on an image plane 5, wherein the curved object place 4 is in close contact with the hemispherical outer surface (the first transmitting surface) 31 of a domed transparent cover (refracting element) 3 of a capsule endoscope. Note, however, that by reversing the optical path taken, the inventive optical system may just as well be used in the form of a projection optical system wherein the planar image plane 5 is projected onto the curved object plane 4 on the hemispherical outer surface 31 of the transparent cover 3.

First of all, the inventive optical system is explained with reference to Example 1 of Fig. 1. In the optical system of Fig. 1, for instance, the inner surface (the second transmitting surface) 32 of the domed transparent cover (a refracting element comprising a transparent medium that is rotationally symmetric about the center axis 1 and has a refractive index of greater than 1) of the capsule endoscope is comprised of a surface that, according to the invention, is concave on the image plane 5 side and has negative power at a position off the center axis 1 and in a section including the center axis 1. Specifically, the inner surface (the second transmitting surface) 32 is comprised of a surface that is obtained by the rotation about the center axis 1 as the axis of rotational symmetry of a curved line located in a section including the center axis 1 (the sheet plane of Fig. 1) and the curved line is defined by an arc having a center at a position off the center axis 1.

And, an imaging optical system 7 located on the image plane 5 side with respect to the transparent cover 3 is made up of an ordinary optical system rotationally symmetric about the center axis 1. On the side of, or in, the transparent cover 3 of the imaging optical system 7, there is an aperture stop 6 located coaxially to the center axis 1.

Such being the arrangement, in a meridional section (the section including the center axis 1), the inner second transmitting surface 32 of the transparent cover 3 has negative refracting power so that a light beam is bent at that second transmitting surface 32 to relay to the imaging optical system 7 a wide angle-of-view image that is not obtained with an ordinary dome shape, thereby forming that image on the planar image plane 5 vertical to the center axis 1. In a sagittal section (that is orthogonal to the meridional section and includes a chief ray 2), the second transmitting surface 32 is concentric to the center axis and, hence, has no refracting power so that the light beam enters the imaging optical system 7 without being bent at the second transmitting surface 32 with the result that a light beam in a 360° - circumferential direction about the center axis 1 is imaged at the image plane 5 without undergoing changes in the azimuth angle.

Further, an object point on the center axis 1, too, may be viewed because of being imaged at a position of the image plane 5 off the center axis. At the center of the image plane 5, light coming from the pointed center portion 33 of the second transmitting surface 32 is imaged; that is, it is preferable not to display that image formed at the image plane 5 on that portion when displaying an image.

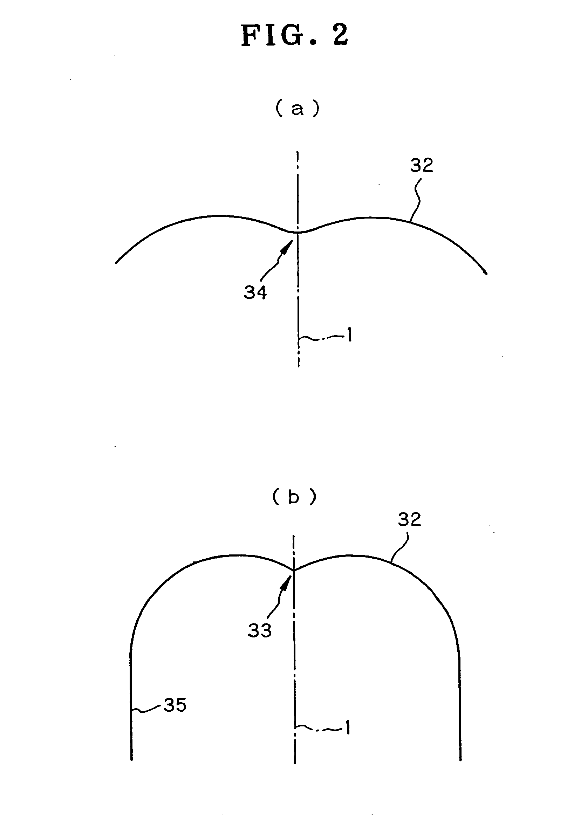

When the pointed portion 33 (the reason of production of pointed portion is that the surface 32 is made from the rotation about the center axis 1 as the axis of rotational symmetry of the arc with the center at a position off the center axis 1) on the center axis 1 offers any problem on processing, the center portion 34 of the inner surface may be compensated for by a smooth curve, as shown in Fig. 2(a). In this case, when displaying the image formed on the image plane 5, it is necessary to make large a non-display area at the center portion. Further by subjecting the center non-display area to image processing, it is easy to display the image as if it were of smooth continuity.

It is more preferable that the peripheral portion of the second transmitting surface 32 having a diameter decreasing toward the center axis 1 is eliminated and, instead, a cylindrical surface 35 is connected to a portion having the maximum diameter for convenient molding of such concave second transmitting surface 32.

It is even more preferable to satisfy the following condition (1):

$$1.1 < R1/(R2+d) \qquad \cdots (1)$$

where R1 is the radius of curvature of the first transmitting surface 31 (curved object plane 4) in the meridional section, R2 is the radius of curvature of the second transmitting surface 32, and d is the minimum thickness between the first transmitting surface 31 and the second transmitting surface 32.

As the lower limit of 1.1 to the above condition (1) is not reached, it will weaken the effect of the domed refracting element 3 on making the angle of view wide.

Tabulated below are the values of R1, R2, d and R1/(R2+d) in Examples 1, 2, 3 and 4 given later.

|  | Example 1 | Example 2 | Example 3 | Example 4 |
|---|---|---|---|---|
| R1 | 5.50 | 5.50 | 20.00 | 5.50 |
| R2 | 2.60 | 2.60 | 2.50 | 2.60 |
| d | 1.00 | 1.00 | 1.50 | 1.00 |
| R1/(R2+d) | 1.53 | 1.53 | 5.00 | 1.53 |

Incidentally, the second transmitting surface 32 has negative power in the meridional section and zero power in the sagittal section; there is astigmatism produced. It is then preferable to correct astigmatism occurring at that surface by use of an aspheric surface located on the image plane 5 side with the stop 6 interposed between them. More preferably, that aspheric surface is located near the image plane 5 because the capability of correcting astigmatism is much more enhanced.

Even more preferably, the surface for correction of astigmatism should be comprised of a rotationally symmetric free-form surface having different powers in the sagittal and meridional sections, and located near the image plane. The rotationally symmetric free-form surface will be referred to later.

As can be seen from the foregoing explanation, if the domed refracting element 3 is attached to the distal end portion of an endoscope, it is then possible to achieve a wide-angle arrangement.

Further, it is possible to round out the first transmitting surface 31 of the refracting element 3 for smoother insertion of the endoscope, as in Example 1.

Alternatively, if such an inventive optical system as shown in Fig. 1 is applied to a capsule endoscope and the domed refracting element 3 is used for part of a transparent cover, it is then possible to achieve a capsule endoscope having a wider angle of view on observation, albeit being of small-format size.

Note here that an image near the center axis 1 becomes a double image; when displaying an image formed on the image plane 5, it is preferable not to display the image near the center axis 1.

It is also noted that an image formed on the concave object plane 4 through such an inventive optical system as described above is formed zonally on the image plane 5, and that if the image formed on the image plane 5 near the center axis 1 is not displayed upon displaying the image, it is then possible to obtain an easy-to-view image.

More preferably, the image on the concave object plane 4 near the center axis 1 is formed near the inner circle of the zonal image formed on the image plane 5 (or an image on the outer periphery of the object plane 4 is formed on the outer periphery of the zonal image). It is then preferable that this inner peripheral image is subjected to image processing to display it as a distorted image of the center point.

The optical system of the invention is now explained more specifically with reference to Examples 1, 2, 3 and 4. The constructional parameters in these examples, which will be given later, have been determined on the results of normal ray tracing from the object plane 4 to the image plane 5 via the transparent cover 3 and the imaging optical system 7, as shown typically in Fig. 1.

For a coordinate system, assume that, in normal ray tracing as shown typically in Fig. 1, the origin is defined by the center of the stop surface 6, the Z-axis positive direction is defined by a direction of the axis of rotational symmetry (the center axis) 1 toward the image plane 5, and the Y-Z plane is defined by the paper plane of Fig. 1. And, the X-axis positive direction is defined by a direction coming in the paper plane and proceeding to rear face of the paper of Fig. 1, and the Y-positive positive direction is defined by an axis that forms with the Y- and Z-axes a right-handed orthogonal coordinate system.

For a decentered surface are the amount of decentration of that surface from the center of the origin of the aforesaid optical system on a coordinate system on which that surface is defined (X, Y and Z are indicative of the X-axis direction, the Y-axis direction, and the Z-axis direction, respectively), and the angles of tilt ($\alpha$, $\beta$, y (°)) of the coordinate systems for defining the surfaces with the centers on the X-, Y- and Z-axes, respectively. In that case, the positive for $\alpha$ and $\beta$ means counterclockwise rotation with respect to the positive directions of the respective axes, and the positive for $\gamma$ means clockwise rotation with respect to the positive direction of the Z-axis. Referring here to how to perform $\alpha$-, $\beta$- and $\gamma$- rotations of the center axis of the surface, the coordinate system that defines each surface is first $\alpha$-rotated counterclockwise about the X-axis of the coordinate system that is defined at the origin of the optical system. Then, the coordinate system is $\beta$-rotated counterclockwise about the Y-axis of the rotated new coordinate system. Finally, the coordinate system is $\gamma$-rotated clockwise about the Z-axis of the rotated new another coordinate system.

When, of optical surfaces forming the optical system of each example, a specific surface and the subsequent surface form together a coaxial optical system, there is a surface spacing given. Besides, the radius of curvature of each surface and the refractive index and Abbe constant of the medium are given as usual.

It is noted that the term with respect to aspheric surfaces on which no data are mentioned in the constructional parameters, given later, is zero. Refractive indices and Abbe constants are given on a d-line (587.56 nm wavelength) basis, and length in mm. The decentration of each surface is given in terms of the amount of decentration from the stop surface, as described above.

In this conjunction, the extended rotation free-form surface is a rotationally symmetric surface given by the following definition.

First, the following curve (b) passing through the origin on the Y-Z coordinate plane is determined.

$$Z = (Y^2/RY) / [1 + \{1 - (C_1 + 1) Y^2/RY^2\}^{1/2}]$$
$$C_2 Y + C_3 Y^2 + C_4 Y^3 + C_5 Y^4 + C_6 Y^5 + C_7 Y^6 + \cdots + C_{21} Y^{20} + \cdots$$
$$C_{n+1} Y^n + \cdots \qquad (b)$$

Then, a curve F(Y) is determined by rotation through an angle $\theta$ (°) of that curve (b) in the X-axis position direction provided that the counterclockwise direction is taken as positive. This curve F(Y), too, passes through the origin on the Y-Z coordinate plane.

First, parallel translation of that curve F(Y) by a distance R in the Y-positive direction (in the Z-negative direction when R is negative), and then rotation of the parallel translated curve about the Z-axis, the extended rotation free-form surface is defined by the obtained rotationally symmetric surface.

As a result, the extended rotation free-form surface becomes a free-form surface (free-form curve) in the Y-Z plane, and a circle with a radius |R| in the X-Y plane.

From this definition, the Z-axis becomes the axis (the axis of rotational symmetry) of the extended rotation free-form surface.

Here, RY is the radius of curvature of the spherical term in the Y-Z section, $C_1$ is a conical constant, and $C_2$, $C_3$, $C_4$, $C_5$, etc. are the aspheric coefficients of first, second, third, and fourth order, respectively.

Example 1

Fig. 1 is illustrative in section of the optical system of Example 1 as taken along its center axis 1 (the axis of rotational symmetry).

The optical system here is built up of a refracting element (transparent cover) 3 formed of a transparent medium that is rotationally symmetric about the center axis 1 and has a refractive index of greater than 1, an imaging optical system 7 that is rotationally symmetric about the center axis 1 and a stop 6 that is located between the refracting element 3 and the imaging optical system 7 coaxially to the center axis 1. The first transmitting surface 31 of the refracting element 3 is comprised of a convex spherical surface having a center lying on the center axis 1, and the second transmitting surface 32 is comprised of a concave toric surface (extended rotation free-form surface) obtained by the rotation about the center axis 1 as the axis of rotational symmetry of a curved line defined by an arc having a center at a position off the center axis 1 in a section including the center axis 1. The second transmitting surface 32 is configured such that a normal to the second transmitting surface 32 makes an angle with the center axis 1 near where the center axis 1 intersects the second transmitting surface 32, and has a pointed portion 33 in a direction toward an image plane 5. And, this optical system has an object plane 4 in alignment with the hemispherical first transmitting surface 31.

The imaging optical system 7 is built up of a plano-convex positive lens L1 and a convex-plano lens L2, and the stop 6 is located just before the plano-convex positive lens L1. And, this optical system has the image plane 5 in alignment with the back surface of a plane-parallel plate 8.

Such being the arrangement, an image on the object plane 4, having a center angle of view of 45° (as measured from the center axis 1) and a wide angle of view in the range of -9° to 85° , is formed on the planar image plane 5 vertical to the center axis 1 via the refracting element 3 rotationally symmetric about the center axis 1, the stop 6 and the imaging optical system 7.

The specifications of Example 1 are:

(Half) angle of view: 94° ,
Angle-of-view range: -9° to 85° (with the center angle of 45° ),
Entrance pupil diameter: 0.27 mm, and
Image size: $\phi$0.14 to $\phi$1.69 mm.

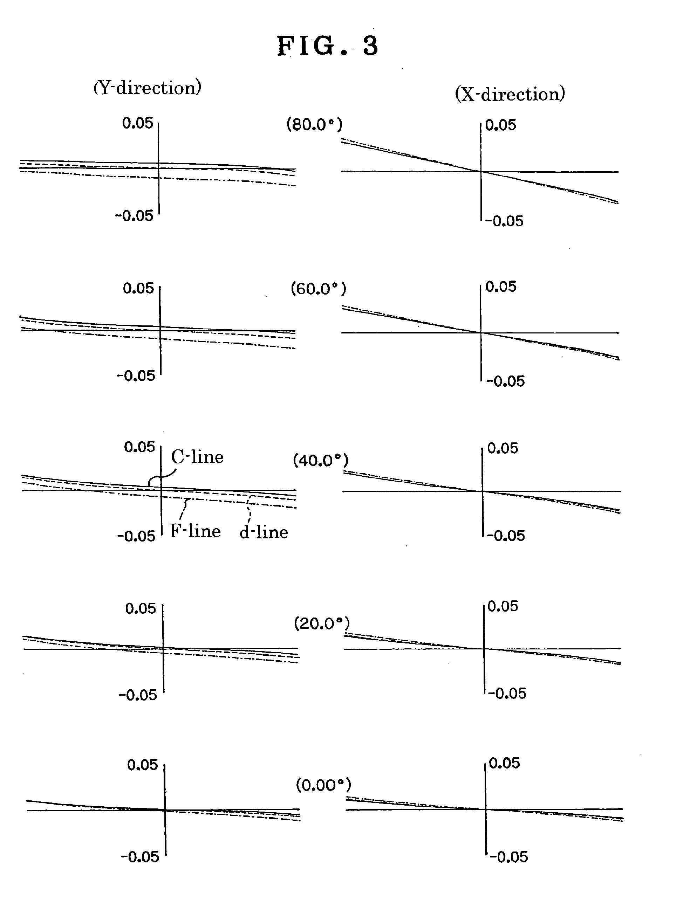

Transverse aberrations of the optical system here are shown in Fig. 3, wherein the bracketed angles at the center stand for angles of view as measured from the center axis 1, and Y (meridional)- and X (sagittal)-direction transverse aberrations at those angles are shown. The same shall apply hereinafter.

Example 2

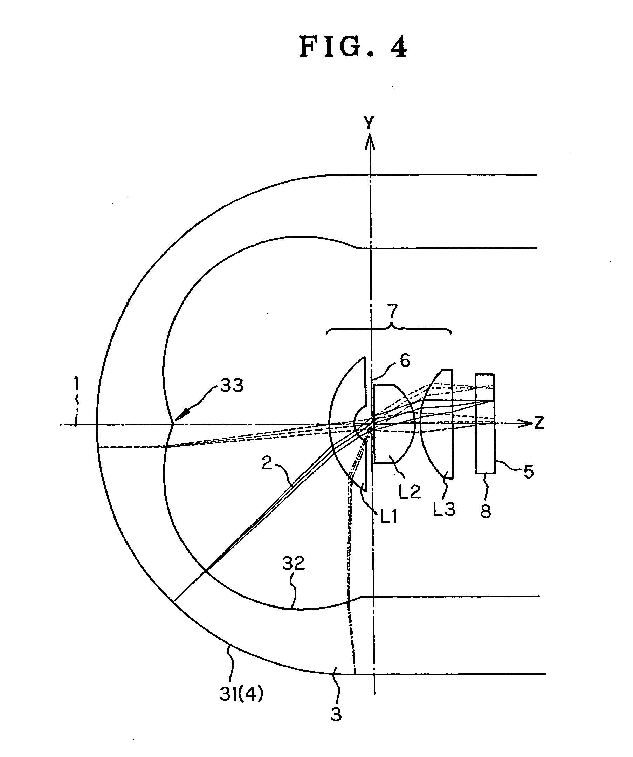

Fig. 4 is illustrative in section of the optical system of Example 2 as taken along its center axis 1 (the axis of rotational symmetry).

The optical system here has an imaging optical system 7 made up of three groups, with a stop 6 located within the imaging optical system 7. More specifically, the optical system is built up of a refracting element (transparent cover) 3 formed of a transparent medium that is rotationally symmetric about the center axis 1 and has a refractive index of greater than 1, the imaging optical system 7 that is rotationally symmetric about the center axis 1, and the stop 6 that is located within the imaging optical system 7 coaxially to the center axis 1. The first transmitting surface 31 of the refracting element 3 is comprised of a convex spherical surface having a center lying on the center axis 1, and the second transmitting surface 32 is comprised of a concave toric surface (extended rotation free-form surface) obtained by the rotation about the center axis 1 or the axis of rotational symmetry of a curved line defined by an arc having a center at a position off the center axis 1 in a section including the center axis 1. The second transmitting surface 32 is configured such that a normal to the second transmitting surface 32 makes an angle with the center axis 1 near where the center axis 1 intersects the second transmitting surface 32, and has a pointed portion 33 in a direction toward an image plane 5. And, this optical system has an object plane 4 in alignment with the hemispherical first transmitting surface 31.

The imaging optical system 7 is built up of a meniscus lens L1 convex on its object side, a plano-convex positive lens L2 and a convex-plano positive lens L3, and the stop 6 is located between the meniscus lens L1 and the plano-convex positive lens L2. And, this optical system has the image plane 5 in alignment with the back surface of a plane-parallel plate 8.

Such being the arrangement, an image on the object plane 4, having a center angle of view of 45° (as measured from the center axis 1) and a wide angle of view in the range of -5° to 85° , is formed on the planar image plane 5 vertical to the center axis 1 via the refracting element 3 rotationally symmetric about the center axis 1, the stop 6 and the imaging optical system 7.

The specifications of Example 2 are:

(Half) angle of view: 90° ,
Angle-of-view range: -5° to 85° (with the center angle of 45° ),
Entrance pupil diameter: 0.21 mm, and
Image size: $\phi$0.10 to $\phi$1.54 mm.

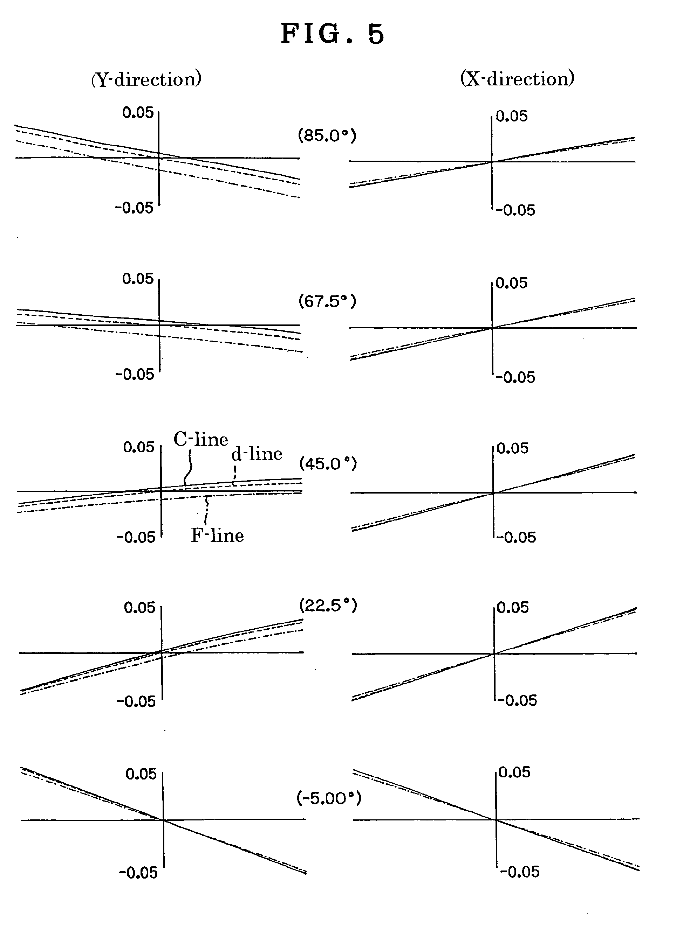

Transverse aberrations of the optical system here are shown in Fig. 5.

Example 3

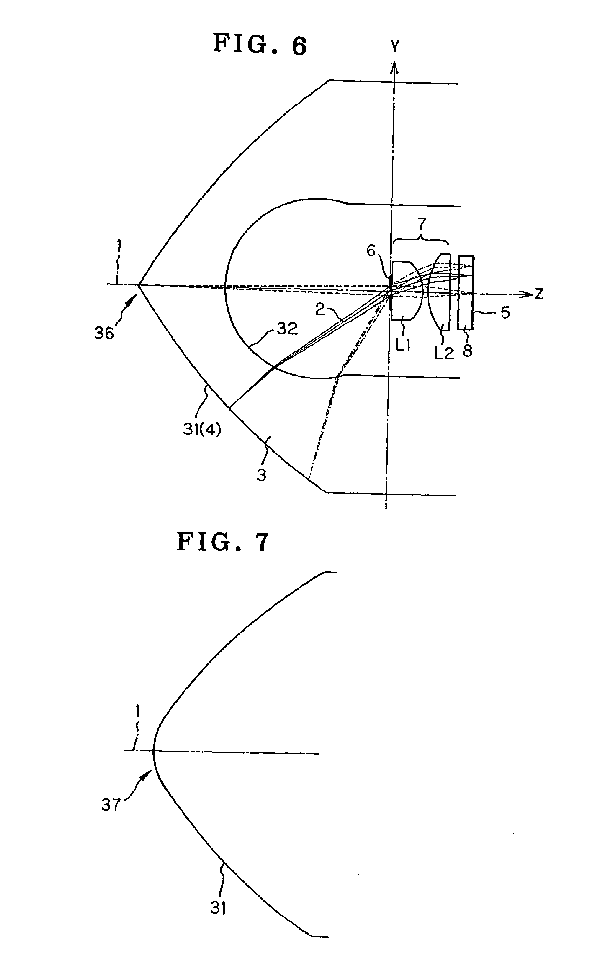

Fig. 6 is illustrative in section of the optical system of Example 3 as taken along its center axis 1 (the axis of rotational symmetry).

In the optical system here, the first transmitting surface 31 of a refracting element (transparent cover) 3 is comprised of an extended rotation free-form surface of pointed shape obtained by the rotation about the center axis 1 or the axis of rotational symmetry of a curved line defined by an arc having a center at a position off the center axis 1 in a section including the center axis 1, and the second transmitting surface 32 is comprised of a concave spherical surface having a center on the center axis 1. More specifically, the optical system is built up of the refracting element (transparent cover) 3 formed of a transparent medium that is rotationally symmetric about the center axis 1 and has a refractive index of greater than 1, an imaging optical system 7 that is rotationally symmetric about the center axis 1 and a stop 6 that is located between the refracting element 3 and the imaging optical system 7 coaxially to the center axis 1. The first transmitting surface 31 of the refracting element 3 is comprised of an extended rotation free-form surface of pointed shape obtained by the rotation about the center axis 1 or the axis of rotational symmetry of a curved line defined by an arc having a center at a position off the center axis 1 in a section including the center axis 1, and the second transmitting surface 32 is comprised of a concave spherical surface having a center on the center axis 1. The first transmitting surface 31 is configured such that a normal to the first transmitting surface 31 makes an angle with the center axis 1 near where the center axis 1 intersects the first transmitting surface 31, and has a pointed portion 36 on an opposite side to an image plane 5. And, this optical system has an object plane 4 in alignment with the first transmitting surface 31 of pointed shape.

The imaging optical system 7 is built up of a plano-convex positive lens L1 and a convex-plano positive lens L2, and the stop 6 is located just before the plano-convex positive lens L1. And, this optical system has the image plane 5 in alignment with the back surface of a plane-parallel plate 8.

Such being the arrangement, an image on the object plane 4, having a center angle of view of 45° (as measured from the center axis 1) and a wide angle of view in the range of 0° to 89.5°, is formed on the planar image plane 5 vertical to the center axis 1 via the refracting element 3 rotationally symmetric about the center axis 1, the stop 6 and the imaging optical system 7.

Note here that the pointed portion 36 of the first transmitting surface 31 on the center axis 1 (the reason of production of pointed portion is that the surface 32 is made from the rotation about the center axis 1 or the axis of rotational symmetry of the arc having a center at a position off the center axis 1) may be complemented by such a smooth curve (curved surface) 37 as shown in Fig. 7.

The specifications of Example 3 are:

(Half) angle of view: 90°,
Angle-of-view range: 0° to 89.5° (with the center angle of 45°),
Entrance pupil diameter: 0.21 mm, and
Image size: $\phi$0.00 to $\phi$1.48 mm.

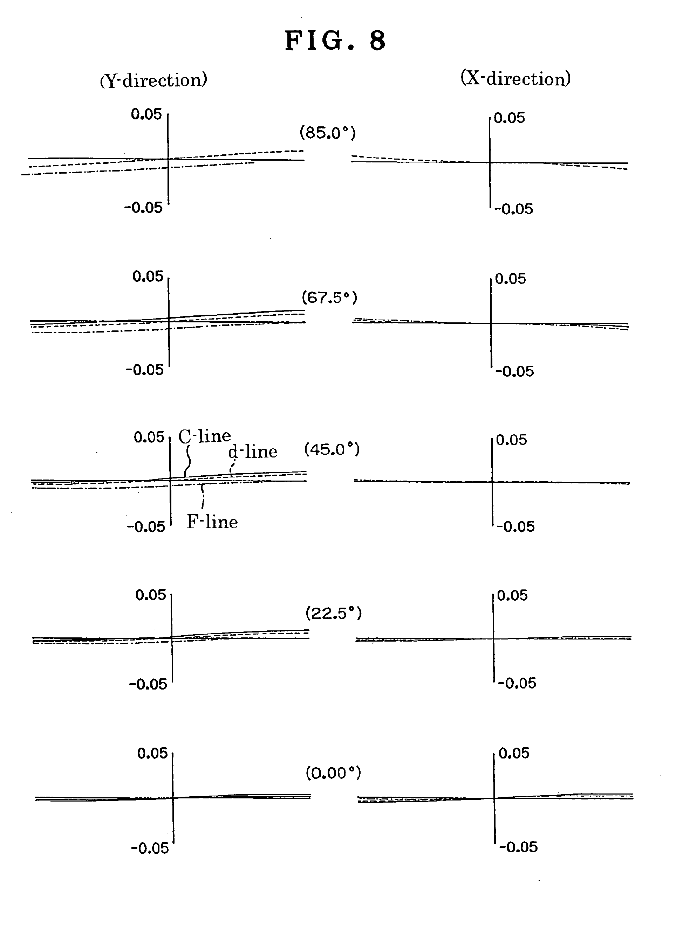

Transverse aberrations of the optical system here are shown in Fig. 8.

Example 4

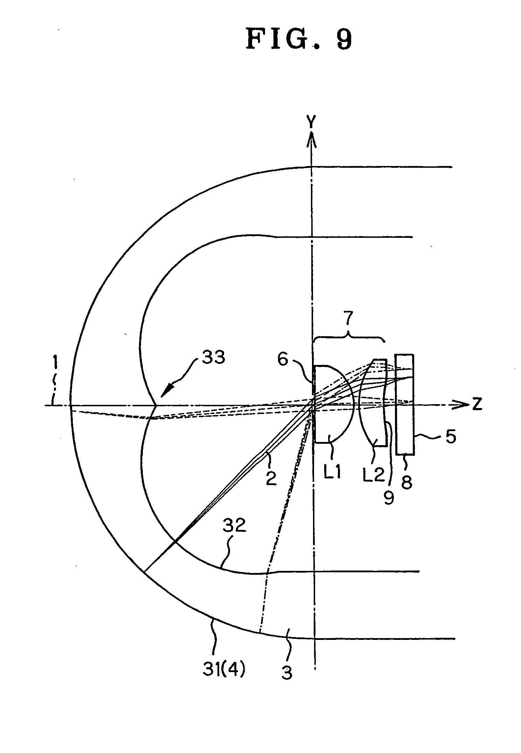

Fig. 9 is illustrative in section of the optical system of Example 4 as taken along its center axis 1 (the axis of rotational symmetry).

In this exemplary optical system with a similar arrangement as in Example 1, astigmatism, etc. occurring at the refracting element 3 in particular are corrected by the introduction in the final surface of an imaging optical system 7 of an extended rotation free-form surface that is rotationally symmetric about the center axis 1. More specifically, the optical system here is built up of a refracting element (transparent cover) 3 formed of a transparent medium that is rotationally symmetric about the center axis 1 and has a refractive index of greater than 1, the imaging optical system 7 that is rotationally symmetric about the center axis 1 and a stop 6 that is located between the refracting element 3 and the imaging optical system 7 coaxially to the center axis 1. The first transmitting surface 31 of the refracting element 3 is comprised of a convex surface having a center lying on the center axis 1, and the second transmitting surface 32 is comprised of a concave toric surface (extended rotation free-form surface) obtained by the rotation about the center axis 1 or the axis of rotational symmetry of a curved line defined by an arc having a center at a position off the center axis 1 in a section including the center axis 1. The second transmitting surface 32 is configured such that a normal to the second transmitting surface 32 makes an angle with the center axis 1 near where the center axis 1 intersects the second transmitting surface 32, and has a pointed portion 33 in a direction toward the image plane 5. And, this optical system has an object plane 4 in alignment with the hemispherical first transmitting surface 31.

The imaging optical system 7 is built up of a plano-convex positive lens L1 and a convex-plano lens L2, and a surface 9 of the convex-plano positive lens 2 on an image plane 5 side is comprised of an extended rotation free-form surface. The stop 6 is located just before the plano-convex positive lens L1. And, this optical system has the image plane 5 in alignment with the back surface of a plane-parallel plate 8.

Such being the arrangement, an image on the object plane 4, having a center angle of view of 45° (as measured from the center axis 1) and a wide angle of view in the range of -9° to 85° , is formed on the planar image plane 5 vertical to the center axis 1 via the refracting element 3 rotationally symmetric about the center axis 1, the stop 6 and the imaging optical system 7.

The specifications of Example 4 are:

(Half) angle of view: 94° ,
Angle-of-view range: -9° to 85° (with the center angle of 45°),
Entrance pupil diameter: 0.27 mm, and
Image size: $\phi$0.17 to $\phi$1.67 mm.

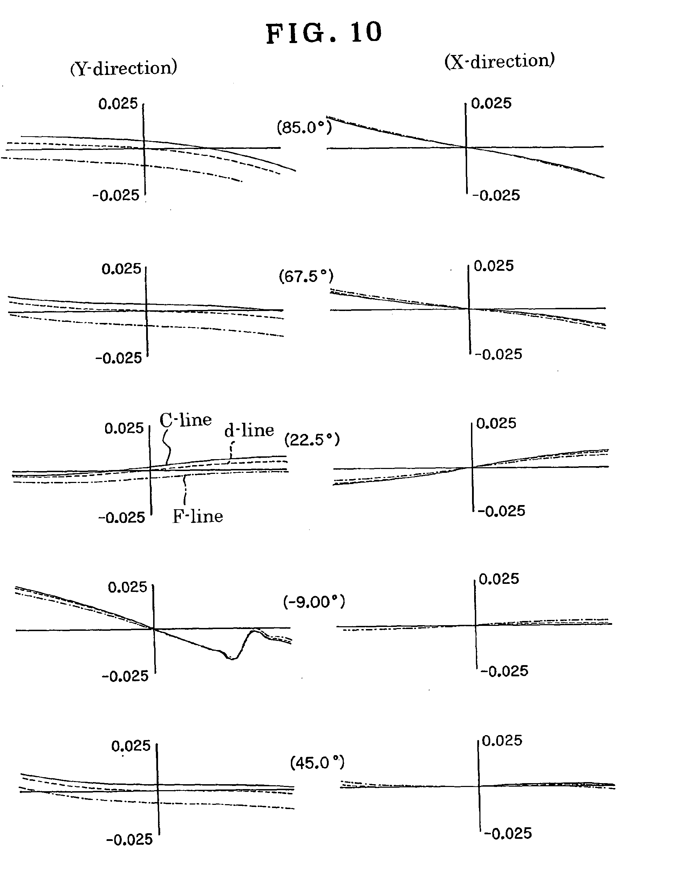

Transverse aberrations of the optical system here are shown in Fig. 10.

The constructional parameters in Examples 1, 2, 3 and 4 are set out below, wherein the acronym "ERFS" indicates an extended rotation free-form surface.

Example 1

| Surface No. | Radius of curvature | Surface separation | Displacement and tilt | Refractive index | Abbe's No. |
|---|---|---|---|---|---|
| Object plane | 5.50 | | (1) | | |
| 1 | 5.50 | | (1) | 1.5163 | 64.1 |
| 2 | ERFS[1] | | (2) | | |
| 3 | ∞ (Stop) | 0.03 | | | |
| 4 | ∞ | 0.90 | | 1.7880 | 47.3 |
| 5 | -1.00 | 0.10 | | | |
| 6 | 1.70 | 0.62 | | 1.7880 | 47.3 |
| 7 | ∞ | 0.33 | | | |
| 8 | ∞ | 0.40 | | 1.5163 | 64.1 |
| Image plane | ∞ | | | | |

ERFS[1]
RY      2.60
θ       45.00
R       -3.18
Displacement and tilt(1)
X       0.00 Y 0.00 Z -5.50
α       0.00 β 0.00 γ 0.00
Displacement and tilt(2)
X       0.00 Y 0.00 Z -3.18
α       0.00 β 0.00 γ 0.00

Example 2

| Surface No. | Radius of curvature 5.50 | Surface separation | Displacement and tilt (1) | Refractive index | Abbe's No. |
|---|---|---|---|---|---|
| Object plane | 5.50 | | (1) | | |
| 1 | 5.50 | | (1) | 1.5163 | 64.1 |

(continued)

| Surface No. | Radius of curvature 5.50 | Surface separation | Displacement and tilt (1) | Refractive index | Abbe's No. |
|---|---|---|---|---|---|
| 2 | ERFS[1] | | (2) | | |
| 3 | 1.80 | 0.50 | (3) | 1.6259 | 35.7 |
| 4 | 0.40 | 0.40 | | | |
| 5 | ∞ (Stop) | 0.03 | | | |
| 6 | ∞ | 0.90 | | 1.7880 | 47.3 |
| 7 | -1.00 | 0.10 | | | |
| 8 | 1.70 | 0.70 | | 1.7880 | 47.3 |
| 9 | ∞ | 0.50 | | | |
| 10 | ∞ | 0.40 | | 1.5163 | 64.1 |
| Image plane | ∞ | | | | |

ERFS[1]

RY    3.00
θ     45.00
R     -3.18
Displacement and tilt(1)
X     0.00 Y 0.00 Z -5. 50
α     0.00 β 0.00 γ 0.00
Displacement and tilt(2)
X     0.00 Y 0.00 Z -3. 18
α     0.00 β 0.00 γ 0.00
Displacement and tilt(3)
X     0.00 Y 0.00 Z -0.90
α     0.00 β 0.00 γ 0.00

Example 3

| Surface No. | Radius of curvature | Surface separation | Displacement and tilt | Refractive index | Abbe's No. |
|---|---|---|---|---|---|
| Object plane | ERFS[1] | | (1) | | |
| 1 | ERFS[1] | | (1) | 1.5163 | 64.1 |
| 2 | 2.50 | | (2) | | |
| 3 | ∞ (Stop) | 0.03 | | | |
| 4 | ∞ | 0.90 | | 1.7880 | 47.3 |
| 5 | -1.00 | 0.10 | | | |
| 6 | 1.70 | 0.62 | | 1.7880 | 47.3 |
| 7 | ∞ | 0.23 | | | |
| 8 | ∞ | 0.40 | | 1.5163 | 64.1 |
| Image plane | ∞ | | | | |

ERFS[1]

RY    20.00
θ     45.00
R     -3. 89
Displacement and tilt(1)
X     0.00 Y 0.00 Z -3.89
α     0.00 β 0.00 γ 0.00
Displacement and tilt(2)

(continued)

| Image plane | ∞ |
|---|---|
| X | 0.00 Y 0.00 Z -4. 50 |
| α | 0.00 β 0.00 γ 0.00 |

Example 4

| Surface No. Object plane | Radius of curvature 5.50 | Surface separation | Displacement and tilt (1) | Refractive index | Abbe's No. |
|---|---|---|---|---|---|
| 1 | 5.50 | | (1) | 1.5163 | 64.1 |
| 2 | ERFS[1] | | (2) | | |
| 3 | ∞ (Stop) | 0.00 | | | |
| 4 | ∞ | 0.90 | | 1.7880 | 47.3 |
| 5 | -1.00 | 0.10 | | | |
| 6 | 1.70 | | | 1.7880 | 47.3 |
| 7 | ERFS[2] | | (3) | | |
| 8 | ∞ | 0.40 | (4) | 1.5163 | 64.1 |
| Image plane | ∞ | | | | |

ERFS[1]

| RY | 2.60 |
|---|---|
| θ | 45.00 |
| R | -3.18 |

ERFS[2]

| RY | -6.48 |
|---|---|
| θ | -5. 06 |
| R | 0.54 |
| C4 | $-2.2094 \times 10^{-1}$ |

Displacement and tilt(1)

| X | 0.00 Y 0.00 Z -5.50 |
|---|---|
| α | 0.00 β 0.00 γ 0.00 |

Displacement and tilt(2)

| X | 0.00 Y 0.00 Z -3.18 |
|---|---|
| α | 0.00 β 0.00 γ 0.00 |

Displacement and tilt(3)

| X | 0.00 Y 0.00 Z 1.62 |
|---|---|
| α | 0.00 β 0.00 γ 0.00 |

Displacement and tilt(4)

| X | 0.00 Y 0.00 Z 1.85 |
|---|---|
| α | 0.00 β 0.00 γ 0.00 |

In Example 1, 2, and 4 mentioned above, the surface obtained by the rotation about the center axis 1 of the curved line defined by an arc has been used for the second transmitting surface 32 of the refracting element 3; however, it is understood that use may be made of an extended rotation free-form surface obtained by the rotation about the center axis 1 of an arc curve having a higher-order term or, alternatively, that surface may be replaced by a curved surface of any desired shape.

By immediate use of the refracting element 3 according to the invention, images having a full 360° - direction angle of view may be taken or projected. However, it is acceptable to cut that refracting element 3 in a section including the center axis 1 into 1/2, 1/3, 2/3, etc. for the purpose of taking or projecting an image having an angle of view of 180°, 120°, 240°, etc. about the center axis 1.

It is here noted that by multiplying the values in the aforesaid numerical examples by a certain coefficient, it is possible to set up an optical system capable of obtaining any desired image height. When the radius of the hemispherical curved object plane 4 of the refracting element 3 is arbitrarily varied, focusing may be implemented by shifting the image plane 5 in the center axis 1 direction, or moving a part, or the whole, of the imaging optical system 7.

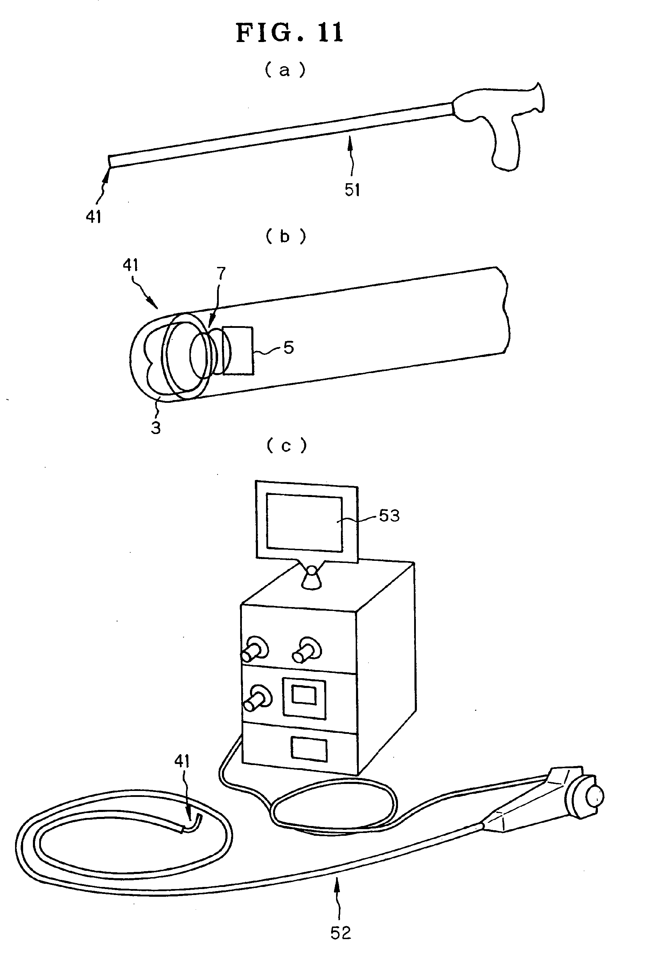

In what follows, an account is given of an example of using a wide angle-of-view taking optical system 41 as an exemplary application of the inventive optical system. Fig. 11 is illustrative of using the inventive wide angle-of-view taking optical system 41 as a taking optical system at the distal end of an endoscope: Fig. 11(a) is illustrative of mounting the inventive wide angle-of-view taking optical system 41 on the distal end 41 of a hard endoscope 51 to take and observe wide angle-of-view images in a full 360°-direction, Fig. 11(b) is illustrative in schematic of the arrangement of that end, and Fig. 11(c) is illustrative of an example of mounting the inventive wide angle-of-view taking optical system 41 on the distal end of a soft electronic endoscope 52 in a similar way to display taken images on a display 53 wherein the taken images are corrected for distortion by imaging processing.

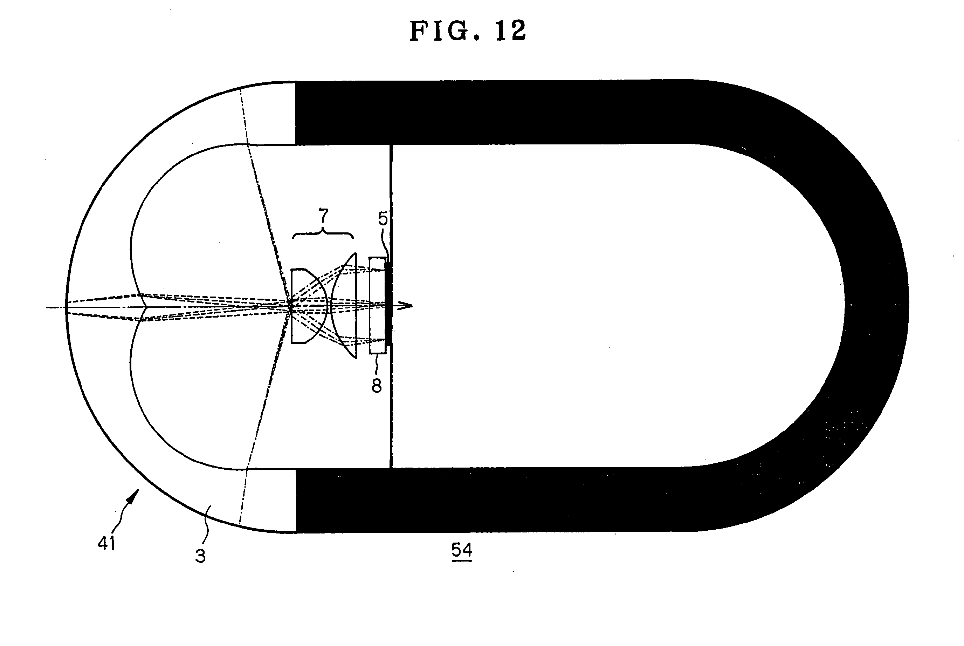

Fig. 12 is illustrative of an example of using the inventive wide angle-of-view taking optical system 41 as a taking optical system for a capsule endoscope 54. With this wide angle-of-view taking optical system 41, it is possible to take and observe full 360°-direction wide angle-of-view images of the intestinal wall or the like in close contact with a hemispherical transparent cover (refracting element) 3 attached over the distal end of the capsule endoscope 54.

APPLICABILITY TO THE INDUSTRY

The invention as described above makes it possible to obtain an optical system that is of small-format size, is well corrected for aberrations, has high resolving power, and is capable of taking or projecting images having a wide angle of view in a simplified arrangement.

**Claims**

1. An optical system which is an imaging system that is to image an object plane having a rotationally symmetric concave shape about a center axis on a planar image plane orthogonal to the center axis and comprises a refracting element formed of a transparent medium including a first transmitting surface rotationally symmetric about the center axis and extending along said object plane and a second transmitting surface rotationally symmetric about the center axis and having a refractive index of greater than 1, wherein a light beam from the object plane is imaged on the image plane through said refracting element, **characterized in that**:

   said second transmitting surface is concave on an image plane side and has negative power at a position off the center axis and in a section including the center axis.

2. The optical system according to claim 1, **characterized in that** a normal to said second transmitting surface makes an angle with the center axis near where the center axis intersects said second transmitting surface.

3. The optical system according to claim 2, **characterized by** further comprising a rear unit that is located on an image plane side with respect to said refracting element, is rotationally symmetric about the center axis and has positive power, wherein a light beam from the object plane is imaged on the image plane via said refracting element and said rear unit in order.

4. The optical system according to claim 3,
   **characterized by** further comprising an aperture that is located coaxially to the center axis between said refracting element and said rear unit or in said rear unit.

5. The optical system according to claim 4, **characterized in that** a portion of an object image formed on the image plane that lies near the center axis is unavailable.

6. The optical system according to claim 5, **characterized in that** an aspheric surface for correction of astigmatism occurring at said refracting element is located on an opposite side thereto with said aperture interposed.

7. The optical system according to claim 5, **characterized in that** said second transmitting surface is comprised of a toric surface rotationally symmetric about the center axis.

8. The optical system according to claim 6, **characterized in that** said second transmitting surface is comprised of a toric surface rotationally symmetric about the center axis.

9. The optical system according to claim 5, **characterized in that** a normal to said first transmitting surface makes an angle with the center axis near where the center axis intersects said first transmitting surface.

10. The optical system according to claim 6, **characterized in that** a normal to said first transmitting surface makes an angle with the center axis near where the center axis intersects said first transmitting surface.

11. The optical system according to claim 5, **characterized in that** said second transmitting surface is comprised of a spherical surface rotationally symmetric about the center axis.

12. The optical system according to claim 6, **characterized in that** said second transmitting surface is comprised of a spherical surface rotationally symmetric about the center axis.

13. An endoscope, **characterized by** comprising an optical system as recited in any one of claims 7 to 12.

14. A capsule endoscope, **characterized by** comprising an optical system as recited in any one of claims 7 to 12, wherein said refracting element is used for part of a domed transparent cover.

15. An optical system as recited in any one of claims 7 to 12, **characterized by** being used as an optical system adapted to project an image located on the image plane onto the object plane.

# FIG. 1

# FIG. 2

( a )

32

34

---1

( b )

32

33

35

---1

# FIG. 3

(Y-direction)                   (X-direction)

# FIG. 4

# FIG. 5

(Y-direction)                              (X-direction)

(85.0°)

(67.5°)

C-line   d-line   (45.0°)

F-line

(22.5°)

(-5.00°)

# FIG. 6

# FIG. 7

# FIG. 8

(Y-direction)                                          (X-direction)

(85.0°)

(67.5°)

C-line
d-line        (45.0°)
F-line

(22.5°)

(0.00°)

# FIG. 9

# FIG. 10

(Y-direction)    (X-direction)

# FIG. 11

## ( a )

41

51

## ( b )

41

7

5

3

## ( c )

53

41

52

FIG. 12

EP 2 037 307 A1

<table>
<tr><td colspan="2" align="center">**INTERNATIONAL SEARCH REPORT**</td><td>International application No.<br>PCT/JP2007/060306</td></tr>
</table>

A. CLASSIFICATION OF SUBJECT MATTER
*G02B13/06*(2006.01)i, *A61B1/00*(2006.01)i, *G02B13/18*(2006.01)i

According to International Patent Classification (IPC) or to both national classification and IPC

B. FIELDS SEARCHED

Minimum documentation searched (classification system followed by classification symbols)
G02B13/06, A61B1/00, G02B13/18

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br><br>A | JP 2001-174713 A (Asahi Optical Co., Ltd.),<br>29 June, 2001 (29.06.01),<br>Full text; all drawings<br>(Family: none) | 1-5,9,11,13,<br>14<br>6-8,10,12,15 |

☐ Further documents are listed in the continuation of Box C.     ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>08 August, 2007 (08.08.07) | Date of mailing of the international search report<br>21 August, 2007 (21.08.07) |
| Name and mailing address of the ISA/<br>Japanese Patent Office | Authorized officer |
| Facsimile No. | Telephone No. |

Form PCT/ISA/210 (second sheet) (April 2005)

**EP 2 037 307 A1**

## REFERENCES CITED IN THE DESCRIPTION

### Patent documents cited in the description

- JP 2001174713 A

- US 5604531 A